(19) **Europäisches Patentamt / European Patent Office / Office européen des brevets**

(11) **EP 4 623 923 B1**

(12) # EUROPEAN PATENT SPECIFICATION

(45) Date of publication and mention
of the grant of the patent:
**11.02.2026 Bulletin 2026/07**

(51) International Patent Classification (IPC):
**A61K 36/31** (2006.01)    **A61P 11/00** (2006.01)
**A61P 31/12** (2006.01)

(21) Application number: **24167421.7**

(22) Date of filing: **28.03.2024**

(52) Cooperative Patent Classification (CPC):
(C-Sets available)
**A61P 11/00; A61K 36/31; A61P 31/12**    (Cont.)

(54) **COMPOSITION COMPRISING TROPAEOLUM MAJUS AND ARMORACIA RUSTICANA**

ZUSAMMENSETZUNG AUS TROPAEOLUM MAJUS UND ARMORACIA RUSTICANA

COMPOSITION COMPRENANT DU TROPAEOLUM MAJUS ET DE L'ARMORACIA RUSTICANA

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**01.10.2025 Bulletin 2025/40**

(73) Proprietor: **Repha GmbH Biologische
Arzneimittel
30855 Langenhagen (DE)**

(72) Inventors:
- **WONNEMANN, Meinolf
30855 Langenhagen (DE)**
- **KASSNER, Nina
30855 Langenhagen (DE)**

(74) Representative: **Fuchs Patentanwälte
Partnerschaft mbB
Tower 185
Friedrich-Ebert-Anlage 35-37
60327 Frankfurt am Main (DE)**

(56) References cited:
- **ALBRECHT UWE ET AL: "A combination of
Tropaeolum majus herb and Armoracia rusticana
root for the treatment of acute bronchitis",
PHYTOMEDICINE, vol. 116, 1 July 2023
(2023-07-01), AMSTERDAM, NL, pages 154838,
XP093207541, ISSN: 0944-7113, DOI: 10.1016/
j.phymed.2023.154838**

- **CHRUBASIK-HAUSMANN SIGRUN:
"Kapuzinerkresse plus Meerrettichwurzel", 2019,
XP093207430, Retrieved from the Internet
<URL:https://www.uniklinik-freiburg.de/
fileadmin/mediapool/08_institute/rechtsmedizin/
pdf/Kapuzinerkresse_Meerrettichwurzel.pdf>
[retrieved on 20240923]**
- **GOOS KARL-HEINZ ET AL: "On-going
investigations on efficacy and safety profile of a
herbal drug containing nasturtium herb and
horseradish root in acute sinusitis, acute
bronchitis and acute urinary tract infection in
children in comparison with other antibiotic
treatments", ARZNEIMITTELFORSCHUNG
DRUG RESEARCH, ECD EDITTIO CANTOR
VERLAG , AULENDORF, DE, vol. 57, no. 4, 1
January 2007 (2007-01-01), pages 238 - 246,
XP001526397, ISSN: 0004-4172**
- **.: "Fachinformation (Zusammenfassung der
Merkmale des Arzneimittels) ANGOCIN Anti-
Infekt N", 1 July 2015 (2015-07-01), Germany,
pages 1 - 3, XP055640574, Retrieved from the
Internet <URL:https://s3.eu-central-1.
amazonaws.com/prod-cerebro-ifap/media_all/
80997.pdf> [retrieved on 20191108]**
- **PERIFERAKIS ARGYRIOS ET AL: "Kaempferol: A
Review of Current Evidence of Its Antiviral
Potential", INTERNATIONAL JOURNAL OF
MOLECULAR SCIENCES, vol. 24, no. 22, 14
November 2023 (2023-11-14), Basel, CH, pages
16299, XP093207524, ISSN: 1422-0067, DOI:
10.3390/ijms242216299**

EP 4 623 923 B1

**(Cont. next page)**

(52) Cooperative Patent Classification (CPC): (Cont.)

C-Sets
**A61K 36/31, A61K 2300/00**

**Description**

The present invention relates to compositions comprising *tropaeolum majus,* or parts thereof, and *armoracia rusticana,* or parts thereof wherein the parts of *tropaeolum majus* in the composition is the herbs in powdered form and the parts of *armoracia rusticana* in the composition is the roots in powdered form; The invention also includes the use of these compositions in methods for the treatment and prevention of RSV-infections; and methods of making such compositions.

Background

RSV-infections (or respiratory syncytial virus infections) are respiratory tract infections caused by the respiratory syncytial virus. RSV is a common virus that can affect people of all ages, but it is a particular concern in infants, young children, and older adults. RSV infections typically lead to mild, cold-like symptoms in healthy adults and older children. However, in infants and individuals with weakened immune systems, such as elderly patients, RSV can cause more severe respiratory symptoms.

Common symptoms of RSV infections include cough, runny or stuffy nose, sneezing, fever, wheezing, difficulty breathing, rapid breathing and decreased appetite.

In infants, RSV infections can lead to bronchiolitis, a condition in which the small airways in the lungs become inflamed and filled with mucus, making it difficult for the child to breathe. Severe cases may require hospitalization.

Each year in the United States, RSV leads to approximately:

- 2.1 million outpatient (non-hospitalization) visits among children younger than 5 years old;

- 58,000-80,000 hospitalizations among children younger than 5 years old;

- 60,000-160,000 hospitalizations among adults 65 years and older;

- 6,000-10,000 deaths among adults 65 years and older; and

- 100-300 deaths in children younger than 5 years old.

RSV is highly contagious and spreads through respiratory droplets when an infected person coughs or sneezes. It can also survive on surfaces for several hours, making it easy to spread through contact with contaminated objects.

There is no specific antiviral treatment for RSV, so management typically involves supportive care to alleviate symptoms, especially in infants and elderly individuals. However, these supportive treatments may have side-effects depending on the specific drug or treatment used. For example, bronchodilators may cause increased heart rate or jitteriness in some individuals.

ALBRECHT UWE ET AL: "A combination of Tropaeolum majus herb and Armoracia rusticana root for the treatment of acute bronchitis", PHYTOMEDICINE, vol. 116, 1 July 2023 (2023-07-01), page 154838, XP093207541, AMSTERDAM, NL ISSN: 0944-7113, DOI: 10.1016/j.phymed.2023.154838 reports that *Tropaeolum majus* and *Armoracia rusticana* root extracts are suitable for a wide range of antibacterial, anti-inflammatory, and immunomodulatory applications in humans.

Chrubasik-Hausmann Sigrun: "Kapuzinerkresse plus Meerrettichwurzel", 2019, XP093207430, reports that *Tropaeolum majus* and *Armoracia rusticana* contain numerous therapeutically active compounds, including various derivatives of kaempferol, which are suitable for a wide range of antibacterial and antiviral applications.

GOOS KARL-HEINZ ET AL: "On-going investigations on efficacy and safety profile of a herbal drug containing nasturtium herb and horseradish root in acute sinusitis, acute bronchitis and acute urinary tract infection in children in comparison with other antibiotic treatments", ARZNEIMITTELFORSCHUNG DRUG RESEARCH, ECD EDITTIO CANTOR VERLAG , AU-LENDOREF, DE, vol. 57, no. 4, 1 January 2007 (2007-01-01), pages 238-246, XP001526397, ISSN: 0004-4172 relates to studies on the efficacy and tolerability of a herbal medicinal product containing nasturtium herb and horseradish in the treatment of acute sinusitis, acute bronchitis, and acute cystitis in children, compared to other antibiotics.

"Fachinformation (Zusammenfassung der Merkmale des Arzneimittels) ANGOCIN Anti-Infekt N", 1 July 2015 (2015-07-01), pages 1-3, XP055640574, relates to the positive effects of ANGOCIN® Anti-Infekt N in urinary tract and respiratory tract infections based on the antibacterial properties of horseradish root and nasturtium herb.

PERIFERAKIS ARGYRIOS ET AL: "Kaempferol: A Review of Current Evidence of Its Antiviral Potential", INTERNATIONAL JOURNAL OF MOLECULAR SCIENCES, vol. 24, no. 22, 14 November 2023, (2023-11-14), page 16299, XP093207524 provides a general overview of which kaempferol derivatives derived from various natural sources may be suitable for which diseases.

Thus, a great need exists for further therapeutic options in treating and preventing RSV infections, particularly for options with reduced risk of severe side effects.

Details of invention

In a first aspect, the invention relates to a composition comprising:

- *tropaeolum majus,* or parts thereof, and

- *armoracia rusticana,* or parts thereof,

  wherein the composition comprises kaempferol-3-rutinoside in an amount of at least 0.020 mg/g, such as at least 0.030 mg/g relative to the weight of the composition,

  wherein

  the parts of *tropaeolum majus* in the composition is the herbs in powdered form and

  the parts of *armoracia rusticana* in the composition is the roots in powdered form.

The first aspect relates to compositions having qualities that contribute to an RSV inhibiting effect of the compositions, particularly upon oral administration to a mammal, such as a human. Hence, this disclosure includes a further aspect.

In the further aspect, the invention relates to a composition of the first aspect comprising *tropaeolum majus,* or parts thereof, and *armoracia rusticana,* or parts thereof, wherein the parts of *tropaeolum majus* in the composition is the herbs in powdered form and the parts of *armoracia rusticana* in the composition is the roots in powdered form for use as a medicament, particularly for use in the treatment or prevention of RSV-infection.

The inventors found that adjusting the manufacturing process of the composition in a way that increases the contents of certain active components can contribute to increasing the composition's ability to inhibit RSV.

In another aspect, the invention relates to a method of making a composition of this disclosure, comprising,

  providing *tropaeolum majus,* or parts thereof,

  wherein

  the parts of *tropaeolum majus* in the composition is the herbs in powdered form,

  providing *armoracia rusticana,* or parts thereof,

  wherein

    the parts of *armoracia rusticana* in the composition is the roots in powdered form.

    subjecting the *armoracia rusticana,* or parts thereof, to a washing procedure,

    wherein the intensity of the washing procedure is adjusted such that the washed *armoracia*

*rusticana,* or parts thereof, comprises a kaempferol-3-rutinoside-content in an amount of at least 0.200 mg/g

and, optionally, wherein the intensity of the washing procedure is such that the microbial quality of the washed *armoracia rusticana,* or parts thereof, represented by a microbial count per g of washed *armoracia rusticana,* or parts thereof, is not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g,

wherein the washing procedure comprises washing with 1-5 liters of washing liquid per 1 kg of *armoracia rusticana,* or parts thereof, at 20-50 rpm in a brush-free front-loaded rotating drum wash machine, for 20-60 minutes, at 10-30°C, at pH 6.5-8.5.

*Tropaeolum majus,* commonly known as nasturtium, is a flowering plant species that is native to South and Central America. It is a popular ornamental plant and is also cultivated for its edible leaves, flowers, and seeds. Historically, nasturtium has been used in traditional medicine for its potential antibacterial properties.

*Armoracia rusticana,* commonly known as horseradish, is a perennial plant belonging to the Brassicaceae family. It is primarily cultivated for its pungent and spicy roots, which are used to make a popular condiment known as horseradish sauce. Horseradish has a long history of use in traditional medicine. It is believed to have potential health benefits due to its antimicrobial properties and the presence of compounds like isothiocyanates. It has been used to alleviate respiratory issues, stimulate digestion, and as a topical treatment for minor aches and pains.

The composition comprises parts of *tropaeolum majus* and parts of *armoracia rusticana,* wherein

  the parts of *tropaeolum majus* in the composition is the herbs, i.e., *herba tropaeoli,* in powdered form, and

  the parts of *armoracia rusticana* in the composition is the roots, i.e., *armoracia rusticanae radix,* in powdered form.

In one embodiment the powder of *tropaeolum majus,* or herbs thereof, and/or the powder of *armoracia rusticana,* or roots thereof, may be made by milling and/or sieving.

The term "parts thereof" is defined herein as parts of the plant which are used for producing the disclosed composition. From *tropaeolum majus* the herbs (such as herbaceous parts which do not lignify, such as flowers, leaves and/or plant stems; *herba tropaeoli*) are used, and from *armoracia rusticana* the roots are used (*armoracia rusticanae radix*).

It has been reported that compositions comprising *tropaeolum majus* and *armoracia rusticana* may have antibacterial activities. However, antiviral activities against RSV have not been analyzed so far. It has been found in the present disclosure that compositions comprising *tropaeolum majus* and *armoracia rusticana* may be suitable for the treatment of RSV-infections.

In one embodiment, a content of kaempferol-3-rutinoside relative to the weight of the *armoracia rusticana,* or roots thereof, (in other words the amount of kaempferol-3-rutinoside in the *armoracia rusticana,* or parts thereof) may be at least 0.200 mg/g, may be at least 0.250 mg/g, may be at least 0.300 mg/g relative to the amount of *armoracia rusticana,* or roots thereof. Optionally, the amount of kaempferol-3-rutinoside in the *armoracia rusticana,* or roots thereof, may be up to 0.800 mg/g, up to 0.750 mg/g, up to 0.700 mg/g. In one embodiment the amount of kaempferol-3-rutinoside in the *armoracia rusticana,* or roots thereof, may be from 0.200 to 0.800 mg/g, from 0.250 to 0.750 mg/g, from 0.275 to 0.700 mg/g, from 0.300 to 0.680 mg/g, or from 0.325 to 0.650 mg/g. In one embodiment the amount of kaempferol-3-rutinoside in the *armoracia rusticana,* or roots thereof, is about $0.620 \pm 0.120$ mg/g, or about $0.375 \pm 0.050$ mg/g. In this context, the *armoracia rusticana,* or roots thereof, are in powdered form. The inventors found that vigorous washing methods will reduce the amount of kaempferol-3-rutinoside in the *armoracia rusticana* and its roots. Adjusting the washing steps to preserve a kaempferol-3-rutinoside amount contributes to a high amount of kaempferol-3-rutinoside in the final composition. As the final composition typically contains further components such as pharmaceutically acceptable excipients, the relative amount of kaempferol-3-rutinoside in the final composition will be lower.

In an embodiment, the composition comprises *armoracia rusticana,* or roots thereof, in powdered form, having a content of kaempferol-3-rutinoside relative to the weight of the *armoracia rusticana,* or roots thereof, from 0.200 to 0.800 mg/g, from 0.250 to 0.750 mg/g, from 0.275 to 0.700 mg/g, from 0.300 to 0.680 mg/g, or from 0.325 to 0.650 mg/g. The composition of this disclosure may be in the form of an oral dosage form, in particular solid oral dosage form, such as a tablet (film-tablet, sublingual tablet, orodispersible tablet, depot-tablet, coated tablet such as an enteric-coated tablet, or sustained release-tablet), a dragee, a capsule (soft capsule, hard capsule, or enteric-coated capsule), pellets, granules, powder and/or printlets. In an embodiment, the composition is a solid oral dosage form, such as a tablet or capsule. In an embodiment, the composition is an immediate release solid oral dosage form. If the composition is a capsule, the capsule may be a gelatine capsule such as a hard gelatine capsule.

The weight amount of *tropaeolum majus,* or herbs thereof, may exceed the weight amount of *armoracia rusticana,* or roots thereof, in the composition. The composition may comprise *tropaeolum majus,* or herbs thereof, and *armoracia rusticana,* or roots thereof, in a weight ratio of from 1.50:1.00 to 5.00:1.00, in some embodiments of about 2.50:1.00. Optionally, this ratio is at least 2.00:1.00, or at least 2.25:1.00. For example, the ratio may range up to 4.00:1.00, or up to 3.00:1.00.

In one embodiment the composition may comprise *tropaeolum majus,* or herbs thereof, in an amount of at least 50 mg/g, at least 60 mg/g, at least 70 mg/g, at least 80 mg/g, at least 90 mg/g, or at least 100 mg/g. Optionally, the composition may comprise *tropaeolum majus,* or herbs thereof, in an amount of at most 500 mg/g, at most 400 mg/g, at most 300 mg/g, or at most 250 mg/g. For example, the composition may comprise *tropaeolum majus,* or herbs thereof, in an amount of from 50 mg/g to 500 mg/g, from 60 mg/g to 400 mg/g, from 90 mg/g to 300 mg/g or from 100 mg/g to 250 mg/g.

In one embodiment the composition may comprise *armoracia rusticana,* or roots thereof, in an amount of at least 25 mg/g, at least 30 mg/g, at least 35 mg/g, or at least 40 mg/g. Optionally, the composition may comprise *armoracia rusticana,* or roots thereof, in an amount of at most 200 mg/g, at most 160 mg/g, at most 130 mg/g, or at most 100 mg/g. For example, the composition may comprise *armoracia rusticana,* or roots thereof, in an amount of from 25 mg/g to 200 mg/g, from 30 mg/g to 160 mg/g, from 35 mg/g to 130 mg/g or from 40 mg/g to 100 mg/g.

In one embodiment the composition may comprise *tropaeolum majus,* or herbs thereof, in an amount of at least 100 mg/g, and/or *armoracia rusticana,* or roots thereof, in an amount of at least 40 mg/g. In yet another embodiment the composition may comprise *tropaeolum majus,* or herbs thereof, in an amount of at most 400 mg/g, and/or *armoracia rusticana,* or roots thereof, in an amount of at most 160 mg/g.

Sufficient amounts of kaempferol-3-rutinoside contribute to achieving a good activity against RSV. In one embodiment the amount of kaempferol-3-rutinoside in the composition may be at least 0.030 mg/g, or at least 0.060 mg/g. Optionally, the amount of kaempferol-3-rutinoside in the composition may be up to 0.300 mg/g, up to 0.250 mg/g, or up to 0.200 mg/g relative to the weight of the composition. In one embodiment the composition may comprise kaempferol-3-ruti-noside in an amount of from 0.020 mg/g to 0.300 mg/g, from 0.025 mg/g to 0.250 mg/g, 0.030 mg/g to 0.200 mg/g, 0.035 mg/g to 0.150 mg/g, 0.040 mg/g to 0.125 mg/g, or 0.050 mg/g to 0.100 mg/g. In one embodiment the amount of kaempferol-3-rutinoside in the composition is about $0.062 \pm 0.010$ mg/g; about $0.062 \pm 0.005$ mg/g; about $0.130 \pm 0.05$ mg/g, or about $0.130 \pm 0.02$ mg/g.

In one embodiment the weight amount of kaempferol-3-rutinoside relative to the weight amount of *armoracia rusticana,* or roots thereof, in the composition may be at least 0.050 mg/g, at least 0.075 mg/g, or at least 0.150 mg/g. Optionally, the amount of kaempferol-3-rutinoside in the composition may be up to 0.750 mg/g, up to 0.625 mg/g, or up to 0.600 mg/g. In one embodiment the composition may comprise kaempferol-3-rutinoside in an amount of from 0.050 mg/g to 0.750 mg/g, from 0.060 mg/g to 0.625 mg/g, 0.075 mg/g to 0.500 mg/g, 0.0875 mg/g to 0.375 mg/g, 0.100 mg/g to 0.3125 mg/g, or 0.125 mg/g to 0.250 mg/g. In one embodiment the amount of kaempferol-3-rutinoside in the composition is about 0.155±0.025 mg/g; about 0.155 ±0.0125 mg/g; about 0.325±0.125 mg/g, or about 0.325 ±0.5 mg/g.

In a useful embodiment, the composition comprises:

> a. *tropaeolum majus,* or herbs thereof, in an amount of from 50 mg/g to 500 mg/g, and

> b. *armoracia rusticana,* or roots thereof, in an amount of from 25 mg/g to 200 mg/g,

wherein a content of kaempferol-3-rutinoside is from 0.020 mg/g to 0.200 mg/g relative to the weight of the composition.

In a useful embodiment, the composition comprises:

> a. *tropaeolum majus,* or herbs thereof, in the form of powdered *tropaeolum majus* herbs in an amount of from 50 mg/g to 500 mg/g, and

> b. *armoracia rusticana,* or roots thereof, in the form of powdered *armoracia rusticana* roots in an amount of from 25 mg/g to 200 mg/g,

wherein a content of kaempferol-3-rutinoside is from 0.050 mg/g to 0.200 mg/g relative to the weight of the composition.

In a useful embodiment, the composition comprises:

> a. *tropaeolum majus,* or herbs thereof, in an amount of from 50 mg/g to 500 mg/g, and

> b. *armoracia rusticana,* or roots thereof, in an amount of from 25 mg/g to 200 mg/g,

wherein a content of kaempferol-3-rutinoside is from 0.200 mg/g to 0.800 mg/g relative to the weight of the *armoracia rusticana,* or roots thereof.

In a useful embodiment, the composition comprises:

> a. *tropaeolum majus,* or herbs thereof, in the form of powdered *tropaeolum majus* herbs in an amount of from 50 mg/g to 500 mg/g, and

> b. *armoracia rusticana,* or roots thereof, in the form of powdered *armoracia rusticana* roots in an amount of from 25 mg/g to 200 mg/g,

wherein a content of kaempferol-3-rutinoside is from 0.250 mg/g to 0.750 mg/g relative to the weight of the *armoracia rusticana,* or roots thereof.

In one embodiment, the composition of this disclosure has a microbial quality represented by a microbial count of aerobic microorganisms (TAMC) per g of composition of not more than 500,000 CFU/g, not more than 400,000 CFU/g, or not more than 300,000 CFU/g. Optionally, the composition of this disclosure has a TAMC per g of composition from 100,000 CFU/g to 500,000 CFU/g, from 200,000 CFU/g to 450,000 CFU/g, from 250,000 CFU/g to 400,000 CFU/g, or from 300,000 CFU/g to 350,000 CFU/g.

In one embodiment, the composition of this disclosure has a microbial quality represented by a microbial count of yeast and mold (TYMC) per g of composition of not more than 50,000 CFU/g, not more than 40,000 CFU/g, or not more than 30,000 CFU/g. Optionally, the composition of this disclosure has a TYMC per g of composition from 10,000 CFU/g to 50,000 CFU/g, from 15,000 CFU/g to 45,000 CFU/g, from 20,000 CFU/g to 40,000 CFU/g, or from 20,000 CFU/g to 35,000 CFU/g.

In one embodiment, the composition of this disclosure represented has a microbial quality represented by a microbial count of bile salt tolerant gram-negative bacteria per g of composition of not more than 10,000 CFU/g, not more than 8,000 CFU/g, or not more than 7,000 CFU/g. Optionally, the composition of this disclosure has a microbial count of bile salt tolerant gram-negative bacteria per g of composition from 5,000 CFU/g to 10,000 CFU/g, from 6,000 CFU/g to 9,500 CFU/g, from 7,000 CFU/g to 9,000 CFU/g, or from 7,5000 CFU/g to 8,500 CFU/g.

In one embodiment the composition has a microbial quality represented by a microbial count of not more than 400,000 CFU total proportion of aerobic microorganisms per g of composition, not more than 40,000 CFU total proportion of yeasts and moulds per g of composition and not more than 8,000 CFU total proportion of bile salt tolerant gram-negative bacteria per g of composition.

Determining "Colony Forming Units" (CFU) is done through performing viable plate counts, also known as the plate dilution or serial dilution method. TAMC, TYMC, E. coli and salmonella are measured according to chapters 2.6.12, 2.6.13 and 2.6.31 of the European Pharmacopoeia (11.5 edition). Bile-tolerant bacteria are measured according to food-safety-method ASU § 64 L05.00-5: 1990-06.

Sample Preparation in general:

• The sample is prepared by dispersing 1 g of sample (such as for example washed *armoracia rusticana* plant parts and/or composition) in sterile saline solution (0.9% NaCl).

Dilution Series:

- The sample is serially diluted (1:1, 1:2, 1:10, 1:20, 1:50, 1:100; 1:1000) to create a range of dilutions, ensuring that the number of microorganisms on a petri dish is countable. This is achieved by mixing samples with sterile saline solution (0.9% NaCl).

Plating:

- The different dilutions are plated onto agar plates. Each dilution is spread on a separate plate to isolate and facilitate counting of colonies.

Incubation:

- The agar plates are incubated at 35°C for 48h.

Counting:

- After incubation, colonies on the agar plates are counted. It is important to select plates where colonies are not too dense to allow for accurate counting. The CFU is then calculated according to below formula taking the dilution into account.

Calculating CFU:

The number of colonies is calculated based on the dilution factors. The formula is:

$$\frac{CFU}{g} = \frac{colonies\ counted}{g\ of\ sample}$$

In one embodiment the composition comprises glucosinolates (GSC). Optionally, the composition comprises glucosinolates comprising glucotropaeolin (GTL), sinigrin, and/or gluconasturtiin (GN). In one embodiment the composition comprises GTL in an amount of at least 3.00 mg/g, at least 3.50 mg/g, at least 4.00 mg/g, at least 4.50 mg/g, at least 5.00 mg/g, or at least 5.50 mg/g. Optionally, the composition comprises GTL in an amount of at most 6.00 mg/g, at most 5.75 mg/g, at most 5.50 mg/g, at most 5.25 mg/g, at most 5.00 mg/g, or at most 4.75 mg/g. Optionally, the composition comprises GTL in an amount from 3.00 mg/g to 6.00 mg/g, or from 3.41 mg/g to 5.23 mg/g.

In one embodiment the composition comprises sinigrin in an amount of at least 4.00 mg/g, at least 4.25 mg/g, at least 4.50 mg/g, at least 4.75 mg/g, at least 5.00 mg/g, at least 5.25 mg/g, at least 5.50 mg/g, or at least 5.75 mg/g. Optionally, the composition comprises sinigrin in an amount of at most 6.00 mg/g, at most 5.75 mg/g, at most 5.50 mg/g, at most 5.25 mg/g, at most 5.00 mg/g, or at most 4.75 mg/g. Optionally, the composition comprises sinigrin in an amount from 4.00 mg/g to 6.00 mg/g, or from 4.05 mg/g to 5.73 mg/g.

In one embodiment the composition comprises GN in an amount of at least 0.50 mg/g, at least 0.75 mg/g, at least 1.00 mg/g, at least 1.25 mg/g, at least 1.50 mg/g, or at least 1.75 mg/g. Optionally, the composition comprises GN in an amount of at most 2.50 mg/g, at most 2.25 mg/g, at most 2.00 mg/g, at most 1.75 mg/g, at most 1.50 mg/g, or at most 1.25 mg/g. Optionally, the composition comprises GN in an amount from 0.50 mg/g to 2.50 mg/g, or from 0.81 mg/g to 1.50 mg/g.

In one embodiment the total amount of glucosinolates in the composition may be at least 7.00 mg/g, at least 7.50 mg/g, at least 8.00 mg/g, at least 8.50 mg/g, at least 9.00 mg/g, or at least 9.10 mg/g. Optionally, the total amount of glucosinolates in the composition may be at most 14.00 mg/g, at most 13.75 mg/g, at most 13.50 mg/g, at most 13.25 mg/g, or at most 13.00 mg/g. Optionally, the total amount of glucosinolates in the composition may be from 7.00 mg/g to 14.00 mg/g, from 8.00 mg/g to 13.50 mg/g, from 9.00 mg/g to 13.00 mg/g, from 9.10 mg/g to 12.00 mg/g, or from 9.21 mg/g to 11.84 mg/g.

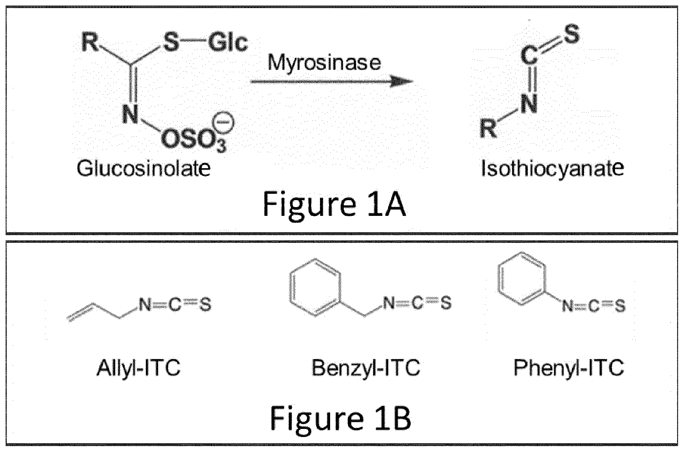

Mustard oils are isothiocyanates (ITCs), which occur as a cleavage product in presence of water and by the enzyme activity of myrosinase (also known as β-thioglucosidase glucohydrolase, EC 3.2.3.1) from their inactive precursors, the glucosides or glucosinolates. In the plant itself, the glucosinolates and myrosinase are separated from each other in different cells. Damage to the plant tissue leads to the abolition of this compartmentalization of the myrosinase and the glucosinolates and the ITCs are formed as cleavage products through hydrolysis (cf. figure 1). This process is also known as the "mustard oil bomb".

All ITCs are characterized by the presence of an N=C=S group with a very electrophilic central carbon atom. This electrophilic carbon atom is considered responsible for the biological effect of ITC, mediated by its reactions with nucleophilic cellular targets. Once released into the epithelial cells of the small or large intestine, ITCs are immediately metabolized in the human organism via conjugation to glutathione followed by the mercapturic acid pathway reaction and finally renal excretion whereas this conjugation leads to ITC inactivation.

In the context of the present disclosure, the composition may release isothiocyanates during or after oral administration. In one embodiment, the ITCs may be selected from allyl-isothiocyanates (A-ITC), benzyl-Isothiocyanates (B-ITC), and/or phenylethyl-/ phenyl-isothiocyanates (PE-ITC). That is because *tropaeolum majus* comprises A-ITC and/or PE-ITC as well as glucosinolate precursors thereof, and *armoracia rusticana* comprises B-ITC as well as glucosinolate precursors thereof. In one embodiment, the allyl-isothiocyanates (A-ITC), benzyl-Isothiocyanates (B-ITC) and phenylethyl-/ phenyl-isothiocyanates (PE-ITC) are released in a subject, optionally after oral administration, in a weight ratio from 3:5:2 to 4:5:1, such as 3:5:2, or 3.5:5:1.5; or 3.5:4.5:1; or 4:5:1. In one embodiment, the allyl-isothiocyanates (A-ITC), benzyl-Isothiocyanates (B-ITC) and phenylethyl-/ phenyl-isothiocyanates (PE-ITC) are released in a subject, optionally after oral administration, in an amount relative to the total amount of ITC-release from 30 to 40 wt.-% (in case of A-ITC), from 40 to 60% (in case of B-ITC), and/or from 10 to 20 wt.-% (in case of PE-ITC).

In one embodiment the composition comprises allyl-isothiocyanates (A-ITC), benzyl-Isothiocyanates (B-ITCs) and phenylethyl-/ phenyl-isothiocyanates (PE-ITC) which are released in a subject in a ratio of between 3:5:2 and 4:5:1 after oral administration of the composition.

Sufficient amounts of ITC contribute to achieving a good activity against RSV.

In one embodiment the composition releases B-ITC as measured in the in-vitro-method as outlined below, in an amount from 0.50 mg/g to 2.50 mg/g, 0.80 mg/g to 2.50 mg/g, 0.81 mg/g to 2.20 mg/g, or from 0.84 mg/g to 2.01 mg/g; and/or the composition releases A-ITC in an amount from 0.20 mg/g to 1.50 mg/g, from 0.25 mg/g to 1.50 mg/g, or from 0.27 mg/g to 1.38 mg/g; and/or the composition releases PE-ITC in an amount from 0.10 mg/g to 1.00 mg/g, from 0.10 mg/g to 0.70 mg/g, or from 0.17 mg/g to 0.51 mg/g relative to the weight of the composition.

The in vitro method for determining the ITC-release from the composition is performed in accordance with Lambrix et al., 2001 *(The arabidopsis epithiospecifier protein promotes the hydrolysis of glucosinolates to nitriles and influences trichoplusia ni herbivory; Lambrix V, Reichelt M, Mitch-ell-Olds T, Kliebenstein DJ and Gershenzon J; The Plant Cell, Vol. 13, 2793-2807, 2001)* which is described in detail below.

Pulverized samples of the composition (50 mg each) are weighed into a centrifuge glass tube and mixed with ascorbic acid (L-(+)-Ascorbinsäure, ACS, >99 %, Thermo Scientific Chemicals) and myrosinase (synonym: thioglucosidase; EC 3.2.1.147; CAS No. 9025-38-1). After adding internal standard (ITC standards are purchased from Fluka, Germany) and water, the tube is quickly sealed with a septum cap, the solution is homogenized by gentle swirling and shaken for 15 minutes at 40°C on a shaker. After the addition of 2 mL of dichloromethane (Dichlormethan, ≥99.5 %, for analysis, stabilized with ethanol, Thermo Scientific® Chemicals) through the septum, the tube is vortexed for 10 sec. and centrifuged at 500g for 5 min. The dichloromethane layer is then removed, dried, and filtered by passage through a column of anhydrous sodium sulfate held with a plug of glass wool in a Pasteur pipette. The remaining aqueous layer is re-extracted with a second 2-mL portion of dichloromethane, and the two extracts were combined and concentrated under nitrogen to 200 μL.

Samples are analyzed by gas chromatography-mass spectrometry (GC-MS) using an Agilent 6890 series gas chromatograph (Agilent Technologies®, Waldbronn, Germany) with an HP5MS column (30-m x 0.25-mm x 0.25 μm film), splitless injection at 200°C, and a temperature program of 35°C for 3 min., a 12°C/min ramp to 96°C, and an 18°C/min ramp to 240°C (with a 6-min final hold).

The recovery of glucosinolate hydrolysis products during the extraction procedure was determined by spiking samples with known amounts of the commercially available hydrolysis products of reference standards of B-ITC, A-ITC, and PE-ITC (ITC standards were purchased from Fluka, Germany).

The composition may comprise one or more binders (binders & adhesives) such as starches (e.g., corn starch and/or pregelatinized starch), cellulose derivatives (such as hydroxypropyl cellulose (HPC), hydroxypropyl methylcellulose (HPMC), and/or microcrystalline cellulose), or povidone (polyvinylpyrrolidone). The one or more binders may be present in an amount of from 10% to 50%, or from 23 % to 28 % by weight of the composition.

The composition may comprise one or more fillers such as lactose, microcrystalline cellulose (MCC), or dicalcium phosphate. The one or more fillers may be present in an amount of from 0.5% to 25%, or from 1 % to 4 % by weight of the composition.

The composition may comprise one or more disintegrants such as croscarmellose sodium, or sodium starch glycolate. The one or more disintegrants may be present in an amount of from 1% to 15%, or from 2 % to 4 % by weight of the composition.

The composition may comprise one or more glidants such as silicon dioxide. The one or more glidants may be present in an amount of from 0.2% to 4%, or from 0.5 % to 1.25 % by weight of the composition.

The composition may comprise one or more lubricants such as magnesium stearate or stearic acid. The one or more lubricants may be present in an amount of from 0.2% to 7%, or from 0.5 % to 1.25 % by weight of the composition.

The composition may comprise one or more colorants such as iron oxide pigments, or titanium dioxide. The one or more colorants may be present in an amount of from 0.1% to 3%, or from 0.75 % to 1.5 % by weight of the composition.

The composition may comprise one or more flavors and/or sweeteners such as mannitol, or sorbitol. The one or more flavors and/or sweeteners may be present in an amount of from 0.1 % to 1.0 % by weight of the composition. In one embodiment the composition is free of flavors and/or sweeteners.

Further, the composition, optionally a tablet, may comprise a coating. The coating may be selected from hydroxypropyl methylcellulose (HPMC), shellac, and polyethylene glycol (PEG), as well as combinations thereof. Optionally, the coating may comprise macrogol 4000, talc, water and a colorant, such as iron oxide pigments. The one or more coatings may be present in an amount of from 1% to 12%, or from 6 % to 9 % by weight of the composition.

The composition may further comprise preservatives and/or antioxidants, such as butylated hydroxyanisole (BHA), butylated hydroxytoluene (BHT), or parabenes. The one or more preservatives and/or antioxidants may be present in an amount of from 0.1 % to 1.0% by weight of the composition. In one embodiment the composition is free of preservatives and/or antioxidants.

In one embodiment the composition may contain fillers, binders and glidants such as cellulose, starch and silicon dioxide. The film-coated finished dosage unit may contain additional film formers, dyes and other components such as hypromellose, iron oxides and hydroxides (E 172) and macrogol. Optionally, the composition may comprise starch, silicium dioxide, cellulose, talc, and stearic acid. In one embodiment the composition may comprise cellulose, iron-oxides and - hydroxides (E 172), hypromellose, potato starch, macrogol, natriumcarboxymethyl-starch, highly disperse silicium dioxide, stearic acid, talcum, and/or titandioxide (E 171).

In a useful embodiment, the composition is a solid oral dosage form such as a tablet or capsule and comprises:

    *a. tropaeolum majus,* or herbs thereof, in an amount of from 50 mg/g to 500 mg/g, and

    *b. armoracia rusticana,* or roots thereof, in an amount of from 25 mg/g to 200 mg/g,

wherein a content of kaempferol-3-rutinoside is from 0.020 mg/g to 0.300 mg/g relative to the weight of the composition.

In a useful embodiment, the composition comprises:

    *a. tropaeolum majus,* or herbs thereof, in the form of powdered *tropaeolum majus* herbs in an amount of from 50 mg/g to 500 mg/g, and

    *b. armoracia rusticana,* or roots thereof, in the form of powdered *armoracia rusticana* roots in an amount of from 25 mg/g to 200 mg/g,

    *c.* one or more binders such as starches, cellulose derivatives or povidone,

    *d.* one or more fillers such as lactose, microcrystalline cellulose or dicalcium phosphate,

wherein a content of kaempferol-3-rutinoside is from 0.020 mg/g to 0.300 mg/g relative to the weight of the composition.

In a useful embodiment, the composition is a solid oral dosage form such as a tablet or capsule and comprises:

    *a. tropaeolum majus,* or herbs thereof, in an amount of from 50 mg/g to 500 mg/g, and

    *b. armoracia rusticana,* or roots thereof, in an amount of from 25 mg/g to 200 mg/g,

wherein a content of kaempferol-3-rutinoside is from 0.200 mg/g to 0.800 mg/g relative to the weight of the *armoracia rusticana,* or roots thereof.

In a useful embodiment, the composition comprises:

    *a. tropaeolum majus,* or herbs thereof, in the form of powdered *tropaeolum majus* herbs in an amount of from 50 mg/g to 500 mg/g, and

    *b. armoracia rusticana,* or roots thereof, in the form of powdered *armoracia rusticana* roots in an amount of from 25 mg/g to 200 mg/g,

    *c.* one or more binders such as starches, cellulose derivatives or povidone,

    *d.* one or more fillers such as lactose, microcrystalline cellulose or dicalcium phosphate,

wherein a content of kaempferol-3-rutinoside is from 0.250 mg/g to 0.750 mg/g relative to the weight of the *armoracia rusticana,* or roots thereof.

In one embodiment the composition of the disclosure may be used as a medicament.

In one embodiment the composition of the disclosure may be used in the treatment or prevention of RSV.

This treatment may be a mono-therapy with the composition only, or in conjunction with other therapies against RSV-infections in order to improve the overall outcome. In this context, "in conjunction" includes administration of the composition before, together or after one or more units of co-medication.

In one embodiment the composition is used for the treatment of a subject, or a patient having been exposed to an RSV-infected person, and/or showing at least one of the symptoms of an RSV-infection selected from fever, cough, runny or stuffy nose, sneezing, sore throat, wheezing, difficult breathing, ear or eye infections, and/or lethargy. In one embodiment the subject or patient has been tested positive for an RSV-infection.

In one embodiment the composition is used for the treatment of a subject or a patient of an age from 2 to 6 years, or from 6 to 12 years, or from 12 to 18 years, or from 18 to 65 years, or above 65 years. In another embodiment the composition is used for the treatment of a subject or a patient who is pregnant, breastfeeding and/or immune-compromised.

In one embodiment the composition is used for the treatment of adults having RSV by administering 4 to 5 unit dosage forms per day. In one embodiment the composition is administered to adults orally with 4 unit dosage forms per day. Children from 12 to 18 years may take the same number of unit dosage forms as adults.

In one embodiment the composition is used for the treatment of adults having RSV by administering the unit dosage forms from 3 to 5 times per day. In one embodiment the composition is administered to adults orally 3 times per day. Children from 12 to 18 years may take the same dose as often as adults.

In one embodiment the composition is used for the treatment of adults having RSV by administering 4 to 5 unit dosage forms and/or from 3 to 5 times per day (from 12 to 25 unit dosage forms per day). In one embodiment the composition is administered to adults orally with 4 unit dosage forms and/or 3 times per day (12 unit dosage forms per day). Children from 12 to 18 years may take the same dose as adults.

In one embodiment the composition is used for the treatment of children from 6 to 12 years having RSV by administering orally with 2 to 4 unit dosage forms per day. In one embodiment the composition is administered to children from 6 to 12 years orally with 3 unit dosage forms per day.

In one embodiment the composition is used for the treatment of children from 6 to 12 years having RSV by administering orally from 3 to 4 times per day. In one embodiment the composition is administered to children from 6 to 12 years orally 3 times per day.

In one embodiment the composition is used for the treatment of children from 6 to 12 years having RSV by administering orally with 2 to 4 unit dosage forms and/or from 3 to 4 times per day (from 6 to 16 unit dosage forms per day). In one embodiment the composition is administered to children from 6 to 12 years orally with 3 unit dosage forms and/or 3 times per day (9 unit dosage forms per day).

In one embodiment the composition is used for the treatment of children below 6 years having RSV by administering orally with 1 to 2 unit dosage forms per day. In one embodiment the composition is administered to children below 6 years orally with 1 unit dosage forms per day.

In one embodiment the composition is used for the treatment of children below 6 years having RSV by administering orally from 1 to 2 times per day. In one embodiment the composition is administered to children below 6 years orally 3 times per day (3 unit dosage forms per day).

In one embodiment the composition is used for the treatment of children below 6 years having RSV by administering orally with 1 to 2 unit dosage forms and/or from 1 to 2 times per day (from 1 to 4 unit dosage forms per day). In one embodiment the composition is administered to children below 6 years orally with 1 unit dosage forms and/or 3 times per day (3 unit dosage forms per day).

The term "unit dosage forms" is defined herein as pharmaceutical formulations that is designed to contain a single dose of a drug product comprising at least 0.020 mg kaempferol-3-rutinoside per single "unit dosage form". In one embodiment one unit dosage form may comprise from 50 to 500 mg herb of *tropaeolum majus* and from 20 to 200 mg roots of *armoracia rusticana.* In one embodiment one unit dosage form may comprise from 100 to 400 mg herb of *tropaeolum majus* and from 40 to 160 mg roots of *armoracia rusticana.* In one embodiment one unit dosage form comprises from 400 to 1500 mg, such as from 500 to 1000 mg of the composition.

In a useful embodiment, the composition is for use in a method of treatment, comprising administering to a subject an effective amount of the composition. An effective amount of the composition may be defined as comprising at least 0.080 mg of kaempferol-3-rutinoside per day in adults and children above 12, at least 0.060 mg of kaempferol-3-rutinoside per day in children between 6 and 12 years, and at least 0.020 mg of kaempferol-3-rutinoside per day in children below 6 years. A preferred mode of administration is oral administration, e.g. in the form of one or more tablets or capsules.

The composition and disclosed production method were developed in order to optimize the amount of *armoracia rusticana* derived kaempferol-3-rutinoside, glucosinolates and mustard oil glycosides without changing other beneficial features of the natural product.

The harvested plant parts are washed prior to further processing. This washing step is important to keep microbiological contamination below the CFU-thresholds in the composition. Sterilization methods, such as UV- or chemical sterilization methods, however, are no valid alternative to washing, since they reduce ITC release of the composition. Thus, in one embodiment the cleaning method does not comprise any sterilization method.

It was found that intensive washing regimes may result in low amounts of kaempferol-3-rutino-side. Thus, a cleaning method was developed which keeps both kaempferol-3-rutinoside above desirable thresholds without creating a need for extensive sterilization. The compositions of this invention may be obtained using alternative washing regimes as long as the effect of intensity on the amount of kaempferol-3-rutinoside is taken into account.

Thus, the intensity of the washing is adjusted such that the washed *armoracia rusticana,* or roots thereof, comprises kaempferol-3-rutinoside-content in an amount of at least 0.200 mg/g. Adjusting the intensity of the washing may include adjusting the length of time, agitation/rotations per minute, temperature, amount of washing liquid and/or pH of the washing liquid.

According to this invention washing is performed with 1-5 liters of washing liquid per 1 kg of *armoracia rusticana,* or parts thereof, at 20-50 rpm in a brush-free front-loaded rotating drum wash machine, for 20-60 minutes, at 10-30°C, at pH 6.5-8.5.

Washing with less than 1 liter washing liquid per 1 kg of *armoracia rusticana,* or roots thereof, may result in too high dirt contamination and/or abrasion, washing with more than 5 liters of washing liquid per 1 kg of *armoracia rusticana,* or roots thereof, may result in a reduction of kaempferol-3-rutinoside content. The rpm of the drum washing machine should not be below 20 rpm (rotations per minute), since otherwise the cleaning process may be incomplete. The rpm of the drum washing machine should not be above 50 in order to maintain the integrity of *armoracia rusticana,* or roots thereof. Washing temperatures below 10°C result in increased microbial load. Washing temperatures above 30°C may reduce kaempferol-3-rutinoside and/or glucosinolates in *armoracia rusticana,* or roots thereof. Washing for less than 20 minutes is not sufficient to separate dirt, washing for more than 60 minutes does not significantly improve cleanliness further, but may reduce kaempferol-3-rutinoside and/or glucosinolates in *armoracia rusticana,* or roots thereof. Washing at a pH below 6.5 may reduce kaempferol-3-rutinoside-content significantly, for example washing at pH 3.5 to 4.5 may result in significantly lower kaempferol-3-ruti-noside contents.

The method of this disclosure may include:

providing *tropaeolum majus,* or herbs thereof,

subjecting the *tropaeolum majus,* or herbs thereof, to a washing procedure,

drying the washed *tropaeolum majus,* or herbs thereof,

milling the dried *tropaeolum majus,* or herbs thereof to obtain a *tropaeolum majus* powder,

providing *armoracia rusticana,* or roots thereof,

subjecting the *armoracia rusticana,* or roots thereof, to a washing procedure,

drying the washed *armoracia rusticana,* or roots thereof,

milling the dried *armoracia rusticana,* or roots thereof to obtain an *armoracia rusticana* powder,

combining the *armoracia rusticana* powder with *tropaeolum majus* and optional excipients,

processing the combination to obtain a composition of this disclosure.

The amount of kaempferol-3-rutinoside in the *armoracia rusticana,* or roots thereof, after the washing procedure is at least 0.200 mg/g. In an embodiment, the amount of kaempferol-3-ruti-noside in the *armoracia rusticana,* or roots thereof, after the washing procedure may be at least 0.250 mg/g, or at least 0.300 mg/g. Optionally, the amount of kaempferol-3-rutinoside in the *armoracia rusticana,* or roots thereof, after the washing procedure may be up to 0.800 mg/g, up to 0.750 mg/g, up to 0.700 mg/g. In one embodiment the amount of kaempferol-3-rutinoside in the *armoracia rusticana,* or roots thereof, after the washing procedure steps may be from 0.200 to 0.800 mg/g, from 0.250 to 0.750 mg/g, from 0.275 to 0.700 mg/g, from 0.300 to 0.680 mg/g, from 0.325 to 0.650 mg/g. In one embodiment the amount of kaempferol-3-rutinoside in the *armoracia rusticana,* or roots thereof, after the washing procedure may be about $0.620 \pm 0.100$ mg/g, or about $0.375 \pm 0.050$ mg/g.

In one embodiment, the *armoracia rusticana,* or roots thereof, after the washing procedure has a microbial quality represented by a microbial count of aerobic microorganisms (TAMC) per g of washed *armoracia rusticana,* or roots thereof, of not more than 300,000 CFU/g, not more than 200,000 CFU/g, or not more than 150,000 CFU/g. Optionally, the *armoracia rusticana,* or roots thereof, has a TAMC per g of washed *armoracia rusticana,* or roots thereof, from 50,000 CFU/g to 300,000 CFU/g, from 60,000 CFU/g to 250,000 CFU/g, from 65,000 CFU/g to 200,000 CFU/g, or from 70,000 CFU/g to 150,000 CFU/g.

In one embodiment, the *armoracia rusticana,* or roots thereof, after the washing procedure has a microbial quality represented by a microbial count of yeast and mold (TYMC) per g of washed *armoracia rusticana,* or roots thereof, of not more than 100,000 CFU/g, not more than 50,000 CFU/g, or not more than 10,000 CFU/g. Optionally, the *armoracia rusticana,* or roots thereof, has a TYMC per g of composition of from 1,000 CFU/g to 100,000 CFU/g, from 1,250 CFU/g to 50,000 CFU/g, from 1,400 CFU/g to 20,000 CFU/g, or from 1,500 CFU/g to 10,000 CFU/g.

In one embodiment, the *armoracia rusticana,* or roots thereof, after the washing procedure has a microbial quality represented by a microbial count of bile salt tolerant gram-negative bacteria per g of washed *armoracia rusticana,* or roots thereof, of not more than 250,000 CFU/g, not more than 100,000 CFU/g, or not more than 25,000 CFU/g. Optionally, the *armoracia rusticana,* or roots thereof, has a total proportion of bile salt tolerant gram-negative bacteria per g of washed *armoracia rusticana* of from 1,000 CFU/g to 250,000 CFU/g, from 2,000 CFU/g to 50,000 CFU/g, from 2,500 CFU/g to 30,000 CFU/g, or from 3,000 CFU/g to 25,000 CFU/g.

In one embodiment the *armoracia rusticana,* or roots thereof, after the washing procedure has a microbial quality represented by a microbial count of not more than 100,000 CFU total proportion of aerobic microorganisms per g of washed *armoracia rusticana,* or roots thereof, not more than 10,000 CFU total proportion of yeasts and moulds per g of washed *armoracia rusticana,* or roots thereof, and not more than 25,000 CFU total proportion of bile salt tolerant gram-negative bacteria of washed *armoracia rusticana,* or roots thereof.

In an alternative embodiment, different charges of *armoracia rusticana,* or roots thereof, washed with other methods, may be mixed to achieve the desired kaempferol-3-rutinoside-contents and CFU-values of microbial load.

It is an advantage of the technology disclosed herein that high amounts of kaempferol-3-rutino-side are safeguarded within a product of natural origin, i.e., containing active ingredients in the form of plants or parts thereof. Addition of extraneous kaempferol-3-rutinoside, glucosinolates and/or mustard oil glycosides to the composition from other sources besides *tropaeolum majus* and *armoracia rusticana* is not a suitable alternative to increase the amounts of kaempferol-3-rutinoside, glucosinolates and/or mustard oil glycosides, since it would change the overall beneficial features of the natural product and mustard oil formation. It would also cause regulatory hurdles. Thus, in one embodiment the composition is free of any kaempferol-3-rutinoside from other sources besides *armoracia rusticana* and/or glucosinolates and/or mustard oil glycosides from other sources besides *tropaeolum majus* and *armoracia rusticana.* In one embodiment, the composition is free of any extracts of kaempferol-3-rutinoside, glucosinolates and/or mustard oil glycosides. In one embodiment, the composition is free of any extracts of *tropaeolum majus* and *armoracia rusticana.* In some embodiments, the composition does not contain plants or parts thereof other than those of *tropaeolum majus* and *armoracia rusticana.*

In another embodiment *tropaeolum majus,* or herbs thereof, and *armoracia rusticana,* or roots thereof, may be dried after harvesting and washing at less than 50°C, at less than 48°C, at less than 45°C, or at less than 42°C. Optionally the drying temperature is from 15°C to 50°C, from 20°C to 48°C, from 25°C to 45°C, or from 35°C to 42°C. The drying time may be from 5 to 100 hours, from 25 to 75 hours, or from 35 to 50 hours. Optionally the drying may be made in a drum dryer.

In one embodiment the *tropaeolum majus,* or herbs thereof, may be dried until a residual moisture is achieved of less than 10.00 wt.-%, of less than 8.00 wt.-%, or of less than 5.00 wt.-%. Optionally, the residual moisture is from 3.00 wt.-% to 15.00 wt.-%, from 4.00 wt.-% to 12.00 wt.-%, or from 5.00 wt.-% to 10.00 wt.-%. *Armoracia rusticana,* or roots thereof, may be dried until a residual moisture is achieved of less than 5.00 wt.-%, of less than 4.00 wt.-%, or of less than 3.00 wt.-%. Optionally, the residual moisture is from 1.00 wt.-% to 8.00 wt.-%, from 1.50 wt.-% to 6.00 wt.-%, or from 2.00 wt.-% to 5.00 wt.-%. The residual moisture may be measured according to chapter 2.2.32 of the European Pharmacopoeia (11.5 edition) or by Karl Fischer titration (DIN ISO 760:1978-12), which is a classic titration method that uses coulometric or volumetric titration to determine trace amounts of water in a sample.

Further, the washed and dried *armoracia rusticana,* or roots thereof, may be stored at room temperature (20°C), or at less than 15°, less than 8°, or less than 4°. Optionally the storage temperature may be from 4°C to 25°C, from 1°C to 15°C, from 2°C to 8°C, or from 2.5°C to 4°C. During the production, the storage time from harvest to preparing the composition may be at most 4 months, at most 5 months, at most 6 months, at most 9 months, or at most 1 year.

The disclosure comprises the following embodiments:

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g, and glucosinolates in an amount of at least 9.00 mg/g relative to the weight of the composition.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g, and glucosinolates in an amount of at least 9.00 mg/g relative to the weight of the composition, wherein the composition is in the form of a tablet.

Some embodiments of this invention include the composition comprising *tropaeolum majus,* or herbs thereof, and *armoracia rusticana,* or roots thereof, wherein the composition comprises kaempferol-3-rutinoside from 0.020 mg/g to 0.300 mg/g and glucosinolates from 9.00 mg/g to 12.00 mg/g.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g, and glucosinolates in an amount of at least 9.00 mg/g relative to the weight of the composition; and wherein *tropaeolum majus,* or herbs thereof, are present in an amount of at least 100 mg/g, and/or *armoracia rusticana,* or roots thereof, are present in an amount of at least 40 mg/g.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g, comprising glucosinolates in an amount of at least 9.00 mg/g relative to the weight of the composition; and wherein *tropaeolum majus,* or herbs thereof, are present in an amount of at least 400 mg/g, and/or *armoracia rusticana,* or roots thereof, are present in an amount of at least 160 mg/g.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, and glucosinolates in an amount of at least 9.00 mg/g relative to the weight of the composition.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, and glucosinolates in an amount of at least 9.00 mg/g relative to the weight of the composition, wherein the composition is in the form of a tablet.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition, wherein the composition is in the form of a tablet or capsule.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition, and comprising glucosinolates in an amount of at least 9.00 mg/g relative to the weight of the composition.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition, having a virus-inhibiting effect on RSV.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition, and comprising glucosinolates in an amount of at least 9.00 mg/g relative to the weight of the composition, having a virus-inhibiting effect on RSV.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, comprising glucosinolates in an amount of at least 9.00 mg/g relative to the weight of the composition, further comprising at least one excipient selected from the list consisting of: fillers, binders, glidants, film formers, and dyes, as well as combinations thereof.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, comprising glucosinolates in an amount of at least 9.00 mg/g relative to the weight of the composition, further comprising at least one excipient selected from the list consisting of: hypromellose, iron oxide, iron hydroxide (E 172), macrogol, starch (such as potato starch and/or sodium carboxymethyl starch), silicon dioxide, cellulose, talc, stearic acid, highly dispersed silicon dioxide, and/or titanium dioxide (E 171), as well as combinations thereof.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition, and comprising glucosinolates in an amount of at least 9.00 mg/g relative to the weight of the composition, having a virus-inhibiting effect on RSV, wherein the composition further comprises fillers, binders and glidants.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition, and comprising glucosinolates in an amount of at least 9.00 mg/g relative to the weight of the composition, having a virus-inhibiting effect on RSV, wherein the composition further comprises cellulose, starch and silicon dioxide.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition, and comprising glucosinolates in an amount of at least 9.00 mg/g relative to the weight of the composition for use as a medicament.

Some embodiments of this invention include the composition comprising *tropaeolum majus,* or herbs thereof, and *armoracia rusticana,* or roots thereof, wherein the composition comprises kaempferol-3-rutinoside in an amount of at least 0.020 mg/g and at most 0.300 mg/g, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition, and comprising glucosinolates in an amount of at least 9.00 mg/g relative to the weight of the composition for use in the prevention and/or treatment of an RSV-infection.

"Kaempferol-3-rutinoside" as used hereinunder is a kaempferol O-glucoside that is kaempferol (i.e. a tetrahydroxyflavone in which the four hydroxy groups are located at positions 3, 5, 7 and 4') attached to a rutinosyl [6-deoxy-alpha-L-mannosyl-(1->6)-beta-D-glucosyl] residue at position 3 via a glycosidic linkage. Its IUPAC-name is: 5,7-dihydroxy-2-(4-hydroxyphenyl)-3-[(2S,3R,4S,5S,6R)-3,4,5-trihydroxy-6-[[(2R,3R,4R,5R,6S)-3,4,5-trihydroxy-6-methyloxan-2-yl]oxymethyl]oxan-2-yl]oxychromen-4-one.

Description of the Figures

Figure 1 - Illustration of the cleavage of glucosinolate and formation of isothiocyanates (ITCs) (simplified representation). [Figure 1 A]: myrosinase hydrolyzes the glycosidic bond between glucose (Glc) and reduced sulfur (S) in the glucosinolate molecule. The ITC, which is also known as mustard oil, is formed via an intermediate. [Figure 1 B]: The residue (R) attached to the ITC determines which ITC it is.

Figure 2 - Bar graph of kaempferol-3-rutinoside-content (K3R) in plant material after different washing protocols. Columns 1-3 show the results based on the washing protocol 1 of the present disclosure, resulting in good CFU-loads, and sufficient K3R-amounts for the treatment or prevention of RSV. Columns 4-5 depict the results after washing protocol 2 as described, resulting in high K3R, but also high CFU-loads. Columns 6-12 the results after washing protocol 3 as described, resulting in low CFU-loads, but also in low K3R.

Figure 3 - Flow chart of production of the tablets according to the disclosure.

Figure 4 - Bar graph of the amounts of kaempferol-3-rutinoside in three compositions 1-3 according to the disclosure.

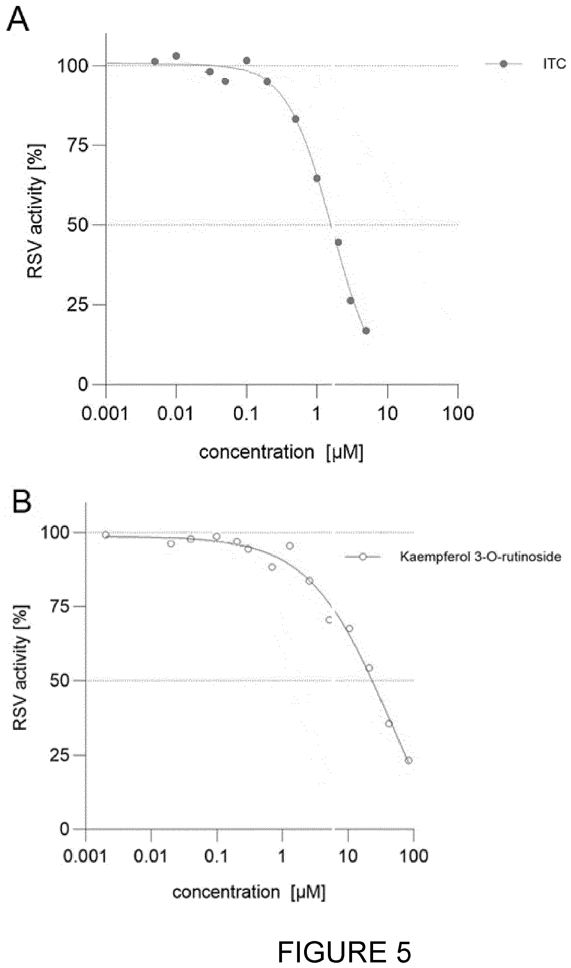

Figure 5 - RSV activity [in %] under addition of increasing ITC (figure 5A) and kaempferol-3-rutinoside (figure 5B) concentrations after infection ("curative setting"), respectively. Data represent means of quadruplicates and triplicates of N=5 independent experiments, respectively.

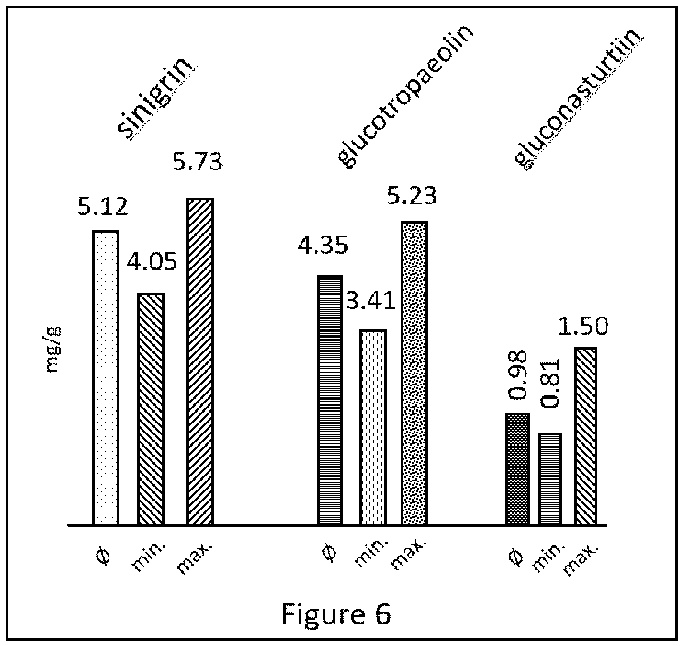

Figure 6 - Bar graph of contents of certain glucosinolates (GSL), i.e. sinigrin, glucotropaeolin, gluconasturtiin in the composition.

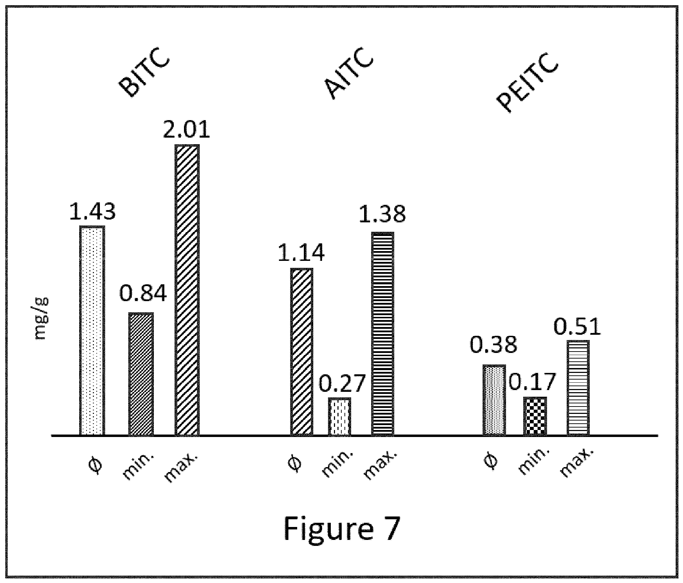

Figure 7 - In vitro release of certain isothyocyanates (ITC) (i.e. B-ITC, A-ITC, PE-ITC) from the composition in mg/g.

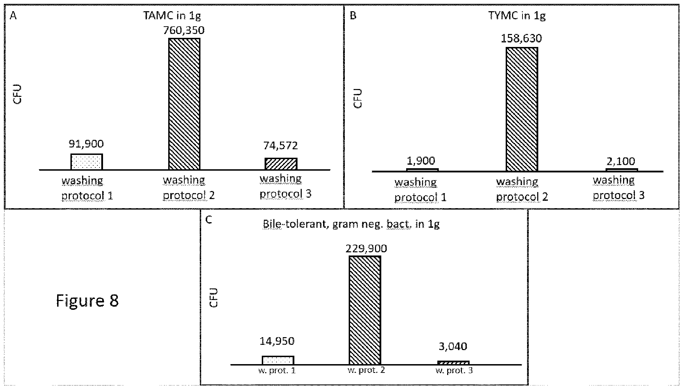

Figure 8 - CFU load measured in washed *armoracia rusticana* after washing protocols 1 - 3 as described herein. The washing protocol 1 as disclosed herein is sufficient to achieve acceptable CFU loads per g of washed *armoracia rusticana.* [Figure 8 A]: Total proportion of aerobic microorganisms (TAMC) per g of washed *armoracia rusticana* according to washing protocols 1 - 3, [Figure 8 B]: Total proportion of yeast and mold (TYMC) per g of washed *armoracia rusticana* according to washing protocols 1 - 3, [Figure 8 C]: Total proportion of bile salt tolerant gram-negative bacteria per g of washed *armoracia rusticana* according to washing protocols 1 - 3.

Examples

### Example 1: Provision of *armoracia rusticana* with kaempferol-3-rutinoside

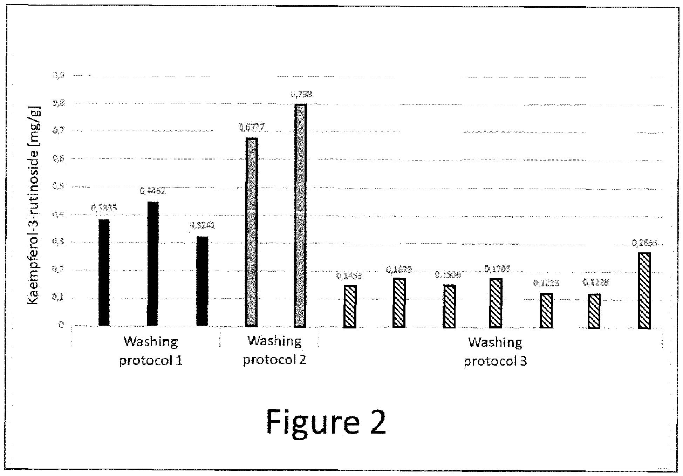

*Armoracia rusticana* was harvested. Different washing protocols were compared with the washing protocol of the disclosure (figure 2).

Protocol 1 comprises washing in a brush-free front-loaded rotating drum wash machine.

Comparative protocol 2 comprises washing in a high-pressure spray washing machine.

Comparative protocol 3 comprises washing in a brush-roller machine combined with immersion in a bio-acetic water solution of 0.1 - 4.0 vol.% + 250 ml of sunflower oil at pH 3.7 to 4.2.

Washing according to protocol 2 resulted in low CFU-loads, but also in low kaempferol-3-rutino-side; washing protocol 3 resulted in high kaempferol-3-rutino-side amounts, but also in inacceptable high CFU-loads due to residual microbial contamination.

Only the washing protocol 1 of the disclosure resulted in acceptable CFU-loads with high amounts of kaempferol-3-rutinoside.

The *armoracia rusticana* roots were cut into cubes of 3-4 cm in order to keep losses as small as possible. Preferred is a direct further processing, if necessary, the cubes can be stored for up to 24 h at about 0°C. The cubes were dried at less than 50°C until a residual moisture of less than 5% and then stored in poly-ethylene-(PE)-bags or paper-bags with inner PE-lining for up to 24 hours.

### Example 2: Provision of plant powder

*Armoracia rusticana,* or parts thereof, were provided cut into cubes of 3-4 cm size, wherein *tropaeolum majus,* or parts thereof, were provided as particles of 0.5-2 cm size.

Both cut plant parts ("cut drug") may be thermically treated (saturated steam sterilization for 1.5 minutes at 94°C) in order to reduce microbial load. Then each of the "cut drug" was milled to a grain size of 300 $\mu$m or less. The milled powder was then further homogenized.

### Example 3: Production of tablet

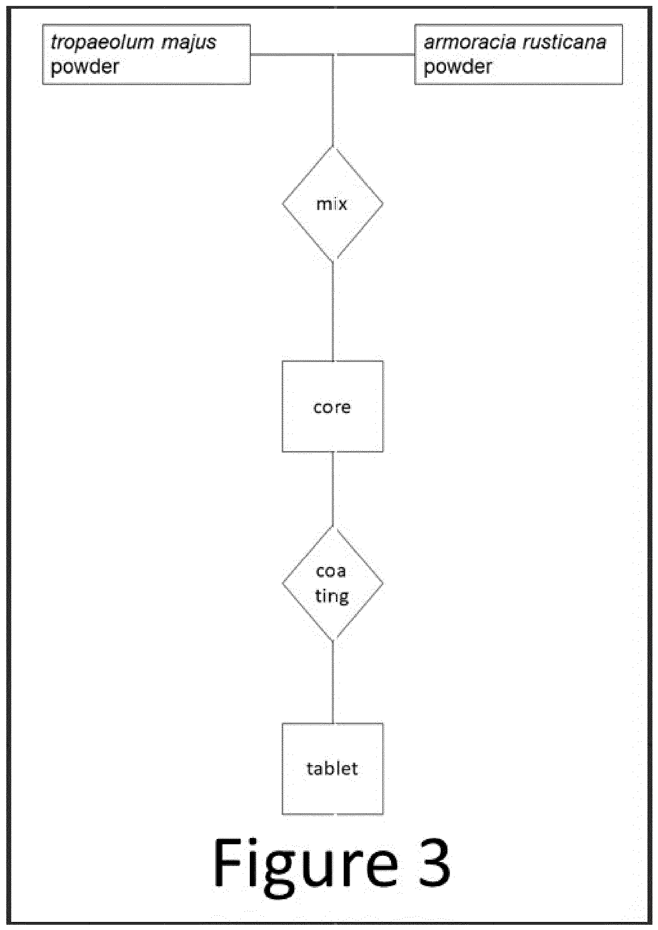

The composition was produced as outlined in figure 3. The powders of the *tropaeolum majus* and *armoracia rusticana* having a grain size of 300 $\mu$m or less were mixed and homogenized with starch and highly disperse silicon dioxide. The mixture was then pressed to obtain the core of the tablet and coated in order to produce the final tablet.

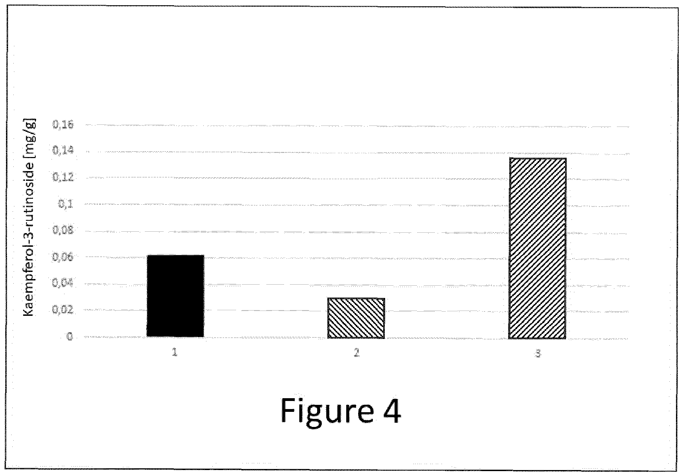

The amount of kempferol-3-rutinoside in the composition was from 0.020 mg/g to 0.140 mg/g (figure 4).

### Example 4: RSV-Test

Effectiveness of isothiocyanates and kaempferol-3-rutinoside against RSV virus was measured in vitro after preceding measures of their individual cytotoxicity on the used cell test system. For testing the anti-viral RSV efficacy of ITCs and kaempferol-3-rutinoside HEp-2 cells were used. HEp-2 is a human hepatoma cell line that is most commonly used in drug metabolism and hepatotoxicity studies. The cells offer high proliferation rates and an epithelial-like morphology that perform many differentiated hepatic functions. To rule out intoxication, the ITC mixture and kaempferol-3-rutinoside were first tested for possible cytotoxicity (MTT assay) in the non-infected cells. Only non-toxic concentrations of the substances were subsequently used for testing antiviral activity.

To investigate the antiviral effectiveness of isothiocyanates, synthetically created mustard oils were used in a high purity in the same ratio as in the disclosed composition: 37.9 vol.-% (v/v) allyl-ITC, 50 vol.-% (v/v) benzyl-ITC and 12.1 vol.-% (v/v) phenyl-ITC. This corresponds approximately to the release of ITC via enzymatic conversion of the glucosinolates in the disclosed composition. Accordingly, 5 $\mu$L BITC, 3.79 $\mu$L AITC, 1.21 $\mu$L PEITC were mixed to a volume fraction of 10 $\mu$L. For the preparation of a 0.01% ITC stock solution, the 10 $\mu$L ITC mixture (100% pure) was dissolved in DMSO by a factor of 100.

To investigate the antiviral effectiveness of kaempferol-3-rutinoside, synthetically created kaempferol-3-rutinoside was used in a purity of > 95.0% (HPLC). 10 mg kaempferol 3-rutinosid (physical state solid), MW 594.52 g/mol, was dissolved in 1 ml ethanol absolute (100% EtOH) ("stock solution"), which corresponded to a molarity of 16.82 mM. For the studies, the ethanolic kaempferol stock solution was diluted with a subsequent 20-fold dilution in medium to a stable kaempferol working solution and a concentration of 500 $\mu$g/mL or 841 $\mu$M with 5% EtOH in medium and used as the starting concentration in the assays. This was followed by serial log2 and log10 gradations in the medium. For the tests of an antiviral effect in the respective scenarios kaempferol was used in the highest concentration of 50 $\mu$g/mL (84 $\mu$M) with a safe 0.5% EtOH in the medium, followed by further log2 and log10 gradations.

In the curative scenario, therapeutic anti-viral activity in plaque reduction assays, with selected infection doses (multiplicity of infection, MOI), was tested via the quantitative analysis of viral plaques (PFU/mL). The addition of the test substances (ITCs or kaempferol-3-rutinosid in $\mu$M) took place after the viral infection (p.i., post infection) and was left on the virus-infected cells until the virus plaques were analyzed. For RSV, a clear dose-dependent reduction of viral plaques was detectable in the therapeutic approach under addition of ITC and kaempferol-3-rutinosid. In the concentration range of 5.0-0.005 $\mu$M ITC, the detected percentage inhibition (%) was between 83.2% and 0.0% RSV.

Moreover, a clear dose-dependent reduction of viral plaques was detectable in the therapeutic approach under addition of kaempferol-3-rutinoside. In the concentration range of 84.0-0.002 μM kaempferol-3-rutinoside, the detected percentage inhibition (%) was between 77% and 0.8% RSV.

## Claims

1. Composition comprising

   *tropaeolum majus,* or parts thereof, and
   *armoracia rusticana,* or parts thereof,
   wherein the composition comprises kaempferol-3-rutinoside in an amount of at least 0.020 mg/g relative to the weight of the composition, wherein
   the parts of *tropaeolum majus* in the composition is the herbs in powdered form and
   the parts of *armoracia rusticana* in the composition is the roots in powdered form.

2. Composition according to claim 1, additionally comprising glucosinolates in an amount of at least 8.00 mg/g relative to the weight of the composition.

3. Composition according to at least one of the previous claims, wherein the composition comprises *tropaeolum majus* herbs in powdered form and/or *armoracia rusticana* roots in powdered form.

4. Composition according to at least one of the previous claims in the form of an oral dosage form, in particular solid oral dosage form, such as a tablet, a dragee, a capsule, pellets, granules, powder and/or printlets.

5. Composition according to at least one of the previous claims, wherein

   *tropaeolum majus,* or parts thereof, are present in an amount of at least 100 mg/g relative to the weight of the composition, and/or
   *armoracia rusticana,* or parts thereof, are present in an amount of at least 40 mg/g relative to the weight of the composition.

6. Composition according to at least one of the previous claims, wherein

   *tropaeolum majus,* or parts thereof, are present in an amount of at most 400 mg/g relative to the weight of the composition, and/or
   *armoracia rusticana,* or parts thereof, are present in an amount of at most 160 mg/g relative to the weight of the composition.

7. Composition according to at least one of the previous claims, having a microbial quality represented by a microbial count of not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g of the composition.

8. Composition according to at least one of the previous claims, wherein the amount of kaempferol-3-rutinoside is at most 0.300 mg/g relative to the weight of the composition.

9. Composition according to at least one of the previous claims, the composition exhibiting a total in vitro ITC-release of at least 1.10 mg/g relative to the weight of the composition.

10. Composition according to at least one of the previous claims, having a specific in vitro ITC-release

    • of A-ITC of at least 0.20 mg/g,
    • of B-ITC of at least 0.80 mg/g,
    • and/or of PE-ITC of at least 0.10 mg/g

    relative to the weight of the composition.

11. Composition according to at least one of the previous claims for use as a medicament.

12. Composition according to at least one of the previous claims for use in the prevention and/or treatment of an RSV-infection.

13. Method of making a composition according to at least one of the preceding claims, comprising,

    providing *tropaeolum majus,* or parts thereof, wherein
    the parts of *tropaeolum majus* in the composition is the herbs in powdered form, providing *armoracia rusticana,* or parts thereof, wherein
    the parts of *armoacia rusticana* in the composition is the roots in powdered form.
    subjecting the *armoracia rusticana,* or parts thereof, to a washing procedure
    wherein the intensity of the washing procedure is adjusted such that the washed *armoracia rusticana,* or parts thereof, comprises kaempferol-3-rutinoside-content in an amount of at least 0.200 mg/g,
    and, optionally, wherein the intensity of the washing procedure is adjusted such that the microbial quality of the washed *armoracia rusticana,* or parts thereof, represented by a microbial count per g of washed *armoracia rusticana,* or parts thereof, is not more than 500,000 CFU total proportion of aerobic microorganisms per g, not more than 50,000 CFU total proportion of yeasts and moulds per gram and/or not more than 10,000 CFU total proportion of bile salt tolerant gram-negative bacteria per g, wherein the washing procedure comprises washing with 1-5 liters of washing liquid per 1 kg of *armoracia rusticana,* or parts thereof, at 20-50 rpm in a brush-free front-loaded rotating drum wash machine, for 20-60 minutes, at 10-30°C, at pH 6.5-8.5.

**Patentansprüche**

1. Zusammensetzung umfassend

   *Tropaeolum majus,* oder Teile davon, und
   *Armoracia rusticana,* oder Teile davon,
   wobei die Zusammensetzung Kämpferol-3-rutinosid in einer Menge von mindestens 0,020 mg/g relativ zum Gewicht der Zusammensetzung umfasst,
   wobei
   die Teile von *Tropaeolum majus* in der Zusammensetzung das Kraut in Pulverform ist und
   die Teile von *Armoracia rusticana* in der Zusammensetzung die Wurzel in Pulverform ist.

2. Zusammensetzung gemäß Anspruch 1, zusätzlich umfassend Glucosinolate in einer Menge von mindestens 8,00 mg/g relativ zum Gewicht der Zusammensetzung.

3. Zusammensetzung gemäß mindestens einem der vorhergehenden Ansprüche, wobei die Zusammensetzung *Tropaeolum majus* Kraut in Pulverform und/oder *Armoracia rusticana* Wurzel in Pulverform umfasst.

4. Zusammensetzung gemäß mindestens einem der vorhergehenden Ansprüche in der Form einer oralen Darreichungsform, insbesondere festen oralen Darreichungsform, wie einer Tablette, eines Dragees, einer Kapsel, Pellets, Granulat, Pulver und/oder Printlets.

5. Zusammensetzung gemäß mindestens einem der vorhergehenden Ansprüche, wobei *Tropaeolum majus,* oder Teile davon, in einer Menge von mindestens 100 mg/g relativ zum Gewicht der Zusammensetzung vorhanden sind, und/oder
   *Armoracia rusticana,* oder Teile davon, in einer Menge von mindestens 40 mg/g relativ zum Gewicht der Zusammensetzung vorhanden sind.

6. Zusammensetzung gemäß mindestens einer der vorhergehenden Ansprüche, wobei *Tropaeolum majus,* oder Teile davon, in einer Menge von höchstens 400 mg/g relativ zum Gewicht der Zusammensetzung vorhanden sind, und/oder
   *Armoracia rusticana,* oder Teile davon, in einer Menge von höchstens 160 mg/g relativ zum Gewicht der Zusammensetzung vorhanden sind.

7. Zusammensetzung gemäß mindestens einem der vorhergehenden Ansprüche, mit einer mikrobiellen Qualität wiedergegeben durch eine Keimzahl von nicht höher als 500.000 KbE Gesamtanteil an aeroben Mikroorganismen pro g, nicht höher als 50.000 KbE Gesamtanteil an Hefen und Schimmelpilzen pro Gramm und/oder nicht höher als 10.000 KbE Gesamtanteil an Gallensalz-toleranten Gram-negativen Bakterien pro g der Zusammensetzung.

8. Zusammensetzung gemäß mindestens einem der vorhergehenden Ansprüche, wobei die Menge an Kämpferol-3-rutinosid höchstens 0,300 mg/g relativ zum Gewicht der Zusammensetzung beträgt.

9. Zusammensetzung gemäß mindestens einem der vorhergehenden Ansprüche, wobei die Zusammensetzung eine in vitro ITC-Gesamtfreisetzung von mindestens 1,10 mg/g relativ zum Gewicht der Zusammensetzung aufweist.

10. Zusammensetzung gemäß mindestens einem der vorhergehenden Ansprüche, mit einer spezifischen in vitro ITC-Freisetzung

    • von A-ITC von mindestens 0,20 mg/g,
    • von B-ITC von mindestens 0,80 mg/g,
    • und/oder von PE-ITC von mindestens 0,10 mg/g

    relativ zum Gewicht der Zusammensetzung.

11. Zusammensetzung gemäß mindestens einem der vorhergehenden Ansprüche zur Verwendung als ein Medikament.

12. Zusammensetzung gemäß mindestens einem der vorhergehenden Ansprüche zur Verwendung in der Vorbeugung und/oder Behandlung einer RSV-Infektion.

13. Verfahren zum Herstellen einer Zusammensetzung gemäß mindestens einem der vorhergehenden Ansprüche, umfassend

    Bereitstellen von *Tropaeolum majus,* oder Teilen davon,
    wobei
    die Teile von *Tropaeolum majus* in der Zusammensetzung das Kraut in Pulverform ist, Bereitstellen von *Armoracia rusticana,* oder Teilen davon,

wobei

die Teile von *Armoracia rusticana* in der Zusammensetzung die Wurzel in Pulverform ist, Unterziehen der *Armoracia rusticana,* oder von Teilen davon, einem Waschverfahren wobei die Intensität des Waschverfahrens so angepasst ist, dass die gewaschene *Armoracia rusticana,* oder Teile davon, einen Gehalt an Kämpferol-3-rutinosid in einer Menge von mindestens 0,200 mg/g umfasst,

und, gegebenenfalls, wobei die Intensität des Waschverfahrens so angepasst ist, dass die mikrobielle Qualität der gewaschenen *Armoracia rusticana,* oder von Teilen davon, wiedergegeben durch eine Keimzahl pro g von gewaschener *Armoracia rusticana,* oder Teilen davon, nicht mehr als 500.000 KbE Gesamtanteil an aeroben Mikroorganismen pro g, nicht mehr als 50.000 KbE Gesamtanteil an Hefen und Schimmelpilzen pro Gramm und/oder nicht mehr als 10.000 KbE Gesamtanteil an Gallensalz-toleranten Gram-negativen Bakterien pro g beträgt,

wobei das Waschverfahren Waschen mit 1-5 Litern Waschflüssigkeit pro 1 kg von *Armoracia rusticana,* oder Teilen davon, bei 20-50 UpM in einer bürstenfreien Frontlader-Waschmaschine mit rotierender Trommel, 20-60 Minuten lang, bei 10-30°C, bei pH-Wert 6,5-8,5 umfasst.

**Revendications**

1. Composition comprenant

du tropaeolum majus, ou des parties de celui-ci, et

de l'armoracia rusticana, ou des parties de celle-ci,

dans laquelle la composition comprend du kaempférol -3-rutinoside en une quantité d'au moins 0,020 mg/g par rapport au poids de la composition,

dans laquelle

les parties de tropaeolum majus dans la composition sont les herbes sous forme de poudre et les parties d'armoracia rusticana dans la composition sont les racines sous forme de poudre.

2. Composition selon la revendication 1, comprenant en outre des glucosinolates en une quantité d'au moins 8,00 mg/g par rapport au poids de la composition.

3. Composition selon au moins l'une des revendications précédentes, dans laquelle la composition comprend des herbes de tropaeolum majus sous forme de poudre et/ou des racines d'armoracia rusticana sous forme de poudre.

4. Composition selon au moins l'une des revendications précédentes sous la forme d'une forme posologique orale, en particulier une forme posologique orale solide, telle qu'un comprimé, une dragée, une capsule, des pellets, des granulés, une poudre et/ou des imprimés.

5. Composition selon au moins l'une des revendications précédentes, dans laquelle

le Tropaeolum majus, ou des parties de celui-ci, sont présents en une quantité d'au moins 100 mg/g par rapport au poids de la composition, et/ou

l'armoracia rusticana, ou des parties de celle-ci, sont présentes en une quantité d'au moins 40 mg/g par rapport au poids de la composition.

6. Composition selon au moins l'une des revendications précédentes, dans laquelle

le tropaeolum majus, ou des parties de celui-ci, sont présents en une quantité maximale de 400 mg/g par rapport au poids de la composition, et/ou

l'armoracia rusticana, ou des parties de celui-ci, sont présents en une quantité maximale de 160 mg/g par rapport au poids de la composition.

7. Composition selon au moins l'une des revendications précédentes, ayant une qualité microbienne représentée par un nombre microbien ne dépassant pas 500 000 UFC proportion totale de micro-organismes aérobies par gramme, ne dépassant pas 50 000 UFC proportion totale de levures et moisissures par gramme et/ou ne dépassant pas 10 000 UFC proportion totale de bactéries Gram négatif tolérantes aux sels biliaires par gramme de composition.

8. Composition selon au moins l'une des revendications précédentes, dans laquelle la quantité de kaempférol-3-rutinoside est au plus de 0,300 mg/g par rapport au poids de la composition.

9. Composition selon au moins l'une des revendications précédentes, la composition présentant une libération totale in vitro d'ITC d'au moins 1,10 mg/g par rapport au poids de la composition.

10. Composition selon au moins l'une des revendications précédentes, ayant une libération spécifique in vitro d'ITC

- d'au moins 0,20 mg/g d'A-ITC,
- d'au moins 0,80 mg/g de B-ITC,
- et/ou d'au moins 0,10 mg/g de PE-ITC

par rapport au poids de la composition.

11. Composition selon au moins l'une des revendications précédentes, destinée à être utilisée comme médicament.

12. Composition selon au moins l'une des revendications précédentes, destinée à être utilisée dans la prévention et/ou le traitement d'une infection par le VRS.

13. Procédé de fabrication d'une composition selon au moins l'une des revendications précédentes, comprenant :

la fourniture de tropaeolum majus, ou de parties de celui-ci,
dans lequel
les parties de tropaeolum majus dans la composition sont les herbes sous forme de poudre, la fourniture d'armoracia rusticana, ou de parties de celui-ci,
dans lequel
les parties d'Armoracia rusticana dans la composition sont les racines sous forme de poudre,
soumettre l'Armoracia rusticana, ou des parties de celle-ci, à une procédure de lavage
dans lequel l'intensité de la procédure de lavage est ajustée de telle sorte que l'Armoracia rusticana lavée, ou des parties de celle-ci, comprenne une teneur en kaempférol-3-rutinoside d'au moins 0,200 mg/g,
et, éventuellement, dans lequel l'intensité du processus de lavage est ajustée de telle sorte que la qualité microbienne de l'armoracia rusticana lavée, ou de parties de celle-ci, représentée par un nombre microbien par gramme d'armoracia rusticana lavée, ou de parties de celle-ci, ne soit pas supérieure à 500 000 UFC proportion totale de micro-organismes aérobies par gramme, pas plus de 50 000 UFC de proportion totale de levures et de moisissures par gramme et/ou pas plus de 10 000 UFC de proportion totale de bactéries gram-négatives tolérantes aux sels biliaires par gramme,
dans lequel la procédure de lavage comprend le lavage avec 1 à 5 litres de liquide de lavage par kg d'armoracia rusticana, ou de parties de celle-ci, à 20-50 tr/min dans une machine à laver à tambour rotatif à chargement frontal sans brosse, pendant 20 à 60 minutes, à une température de 10 à 30 °C, à un pH de 6,5 à 8,5.

Figure 1A

Figure 1B

Figure 2

Figure 3

Figure 4

FIGURE 5

Figure 6

Figure 7

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALBRECHT UWE et al.** A combination of Tropaeolum majus herb and Armoracia rusticana root for the treatment of acute bronchitis. *PHYTOMEDICINE*, 01 July 2023, vol. 116, ISSN 0944-7113, 154838
- **CHRUBASIK-HAUSMANN SIGRUN**. *Kapuzinerkresse plus Meerrettichwurzel*, 2019
- On-going investigations on efficacy and safety profile of a herbal drug containing nasturtium herb and horseradish root in acute sinusitis, acute bronchitis and acute urinary tract infection in children in comparison with other antibiotic treatments. **GOOS KARL-HEINZ et al.** ARZNEIMITTELFORSCHUNG DRUG RESEARCH. ECD EDITTIO CANTOR VERLAG, 01 January 2007, vol. 57, 238-246
- *Fachinformation (Zusammenfassung der Merkmale des Arzneimittels) ANGOCIN Anti-Infekt N*, 01 July 2015, 1-3
- **PERIFERAKIS ARGYRIOS et al.** Kaempferol: A Review of Current Evidence of Its Antiviral Potential. *INTERNATIONAL JOURNAL OF MOLECULAR SCIENCES*, 14 November 2023, vol. 24 (22), 16299
- *CHEMICAL ABSTRACTS*, 9025-38-1